# EUROPEAN PATENT SPECIFICATION

(11) **EP 3 028 696 B1**
(45) Date of publication and mention of the grant of the patent: **27.01.2021**
(21) Application number: 14831281.2
(22) Date of filing: 01.07.2014
(51) Int. Cl.: A61K 31/198, A61P 35/00, A61P 39/00, A61K 9/20, A61P 1/02, A61P 1/08, A61P 1/10, A61P 43/00, A61K 9/00

(54) **AGENT FOR ALLEVIATING SIDE EFFECTS IN CANCER CHEMOTHERAPY**
MITTEL ZUR LINDERUNG DER NEBENWIRKUNGEN EINER CHEMOTHERAPIE BEI KREBS
AGENT PERMETTANT D'ATTÉNUER LES EFFETS SECONDAIRES LORS DE TRAITEMENT DU CANCER PAR CHIMIOTHÉRAPIE

(30) Priority: 31.07.2013 JP 2013159736
(43) Date of publication of application: 08.06.2016
(73) Proprietor: AJINOMOTO CO., INC., Chuo-ku Tokyo 104-8315 (JP)
(72) Inventor: KURIHARA, Shigekazu, Kawasaki-shi Kanagawa 210-8681 (JP); TSUCHIYA, Takashi, Sendai-shi Miyagi 983-0824 (JP)
(74) Representative: Mewburn Ellis LLP
(86) International application number: PCT/JP2014/067587
(87) International publication number: WO 2015/015989

(56) References cited:
- EP-A1- 1 486 208
- EP-A1- 1 767 200
- JP-A- 2003 531 164
- JP-A- 2004 513 957
- JP-A- 2012 502 997
- US-A1- 2007 141 175
- MICHI HATSUNO ET AL.: 'N-Acetylcysteine ni yoru 5-Fluorouracil Yudosei Kotsuzui Dokusei no Keigen' THE PHARMACEUTICAL SOCIETY OF JAPAN DAI 130 NENKAI YOSHISHU 3 05 March 2010, page 155, XP008182722
- YUKI KANEMATSU ET AL.: '1. FOLFOX no Massho Shinkei Shogai ni Taisuru Yobosaku no Jittai Chosa' AICHI PREFECTURAL JOURNAL OF HOSPITAL PHARMACY vol. 36, no. 3, 2008, pages 47 - 50, XP008182235
- YASUYUKI SAZUKA ET AL.: 'Glutamate Transporter Mediated Increase of Antitumor Activity by Theanine, an Amino Acid in Green Tea' YAKUGAKU ZASSHI vol. 122, no. 11, 2002, pages 995 - 999, XP008182115
- CHIEKO INOUE ET AL.: 'Ryokucha Amino San theanine ni yoru Ko-Shuyozai Fukusayo Keigen Mechanism' PROCEEDINGS OF THE JAPANESE CANCER ASSOCIATION vol. 63 RD, 2004, page 463, XP008183217
- YASUYUKI SADZUKA ET AL.: 'Efficacies of tea components on doxorubicin induced antitumor activity and reversal of multidrug resistance' TOXICOLOGY LETTERS vol. 114, 2000, pages 155 - 162, XP000925751
- TETSURO SHIBAKUSA ET AL.: 'Shinshu-ka deno Seitai Hanno to Taisha Dotai' Mouse Geka Shujutsu Model ni Okeru Amino San Cystine Theanine Jutsuzen Toyo ni yoru Jutsugo Ika Koshin no Yokusei Koka' THE JAPANESE JOURNAL OF SURGICAL METABOLISM AND NUTRITION vol. 47, no. 1, February 2013, pages 25 - 30, XP008182116
- TOMOYUKI MORIYA ET AL.: 'B. Amino San Toyo ni yoru Taisha Seitai no Shushoku 3. Shinshu-ji no Amino San Toyo: Shitsu to Ryo, Dochira ga Juyo ka?' THE JAPANESE JOURNAL OF SURGICAL METABOLISM AND NUTRITION vol. 46, no. 2, 2012, pages 41 - 48, XP008182120
- SHIGEKAZU KURIHARA ET AL.: 'Enhancement of Antigen-Specific Immunoglobulin G Production in Mice by Co-Administration of L-Cystine and L- Theanine' JAPANESE SOCIETY OF VETERINARY SCIENCE vol. 69, no. 12, 2007, pages 1263 - 1270, XP055313174

## Description

### Technical Field

The present invention relates to an agent for reducing side effects of cancer chemotherapy. The present invention also relates to an agent for improving the treatment completion rate of cancer chemotherapy, and a prophylactic and/or therapeutic agent for cancer.

### Background Art

In general, cancer is uncontrollable abnormal proliferation of cells caused by mutation of DNA, and characteristically shows a faster proliferation rate than normal cells. Chemotherapeutic agents utilize the fast proliferation rate and cause damage on the actively dividing target cancer cells by inhibiting the cell division. However, this action is not an action specific to cancer cells, and influences normal cells as well. As a result, secondary reactions associated with the administration of chemotherapeutic agents, what is called side effects, are developed at various regions.

Cancer chemotherapy includes some methods as treatment forms. In combined modality chemotherapy, radiation therapy, surgical operation and the like are performed in combination with drugs. A multiple drug therapy (combination chemotherapy) is a method of simultaneously administering many drugs to patients, and drugs having different action mechanisms and different side effects are selected. This method is advantageous in that, different from a treatment with a single drug, possibility of cancer acquiring resistance to chemotherapeutic agents can be minimized. A (postoperative) adjuvant chemotherapy (adjuvant chemotherapy) is performed for a given period after removal of cancer by surgical operation and the like to suppress the recurrence risk of cancer. Since metastatic cancer cells newly proliferate and divide actively, the adjuvant chemotherapy is often effective. A preoperative chemotherapy (neoadjuvant chemotherapy) is mainly performed for breast cancer, and aims to surgically remove cancer after making the tumor small by chemotherapy before the surgery.

While cancer chemotherapy is effective for cancer cells that divide actively, the living body also contains normal tissues and cells showing fast metabolism and active division like cancer cells. Chemotherapeutic agents cause serious side effects in some cases since they also act on such normal tissues and cells showing active division. In such cases, chemotherapy is difficult to continue, and the treatment may be stopped or the dose of anticancer agent may be decreased. Therefore, the treatment effects of chemotherapy (shrinkage of cancer, lowering of the risk of cancer recurrence and the like) cannot be obtained sufficiently.

The side effects of chemotherapeutic agents are defined in Common Terminology Criteria for Adverse Events v4.0 (CTCAE) including the grades of symptoms. While the expression of each symptom varies in the level of strength depending on the kind of chemotherapeutic agent, the main adverse events include anorexia, nausea, vomiting, diarrhea, stomatitis, bone marrow toxicity (leukocyte, granulocytopenia), skin disorder, hair loss, neuropathy, interstitial pneumonia, hepatopathy, renopathy, cardiac disorder and the like. To obtain maximum effects of chemotherapy, suppression of these side effects is an important issue.

At present, some supporting therapies to suppress side effects have been performed. Examples of a supporting therapy for infections due to granulocytopenia associated with bone marrow toxicity include a method including prophylactic administration of an ST combination agent for preventing infection (combination agent of sulfamethoxazole (sulfa drug) and trimethoprim (antibacterial agent)), a method using an antifungal syrup and the like.

A supporting therapy for bone marrow toxicity includes compensating for a decrease in red blood cells and platelets by transfusion, and by administration of synthetic G-CSF (granulocyte-colony stimulating factor) in granulocytopenia. In some cases where severe bone marrow toxicity is developed, hematopoietic stem cells are destroyed. In this event, hematopoietic stem cells collected from patients prior to the chemotherapy are cultured, and injected by autotransplantation after the chemotherapy.

As a supporting therapy for gastrointestinal symptoms, various supporting therapies have been performed for stomatitis, vomiting, and diarrhea as mentioned below. Since stomatitis easily becomes intractable by mucosal disorder due to chemotherapy and infection, oral care is performed as a supporting therapy, and measures are taken such as avoiding ingestion of extremely hot things and the like. Vomiting is controlled by the vomiting central nervous system present in medulla oblongata. There are three mechanisms proposed for vomiting due to chemotherapy, and vomiting is basically classified by when it was developed, and a centrally acting drug is used for each. Diarrhea includes premature diarrhea due to the stimulation of autonomic nerve, which emerges on the day of chemotherapy, and tardive diarrhea associated with mucosal disorder of the gastrointestinal tract, which occurs in several days to about 2 weeks after the treatment. Antidiarrheal drugs are often used for them.

These supporting therapies are all symptomatic therapies, which are applied after the onset of side effects, and do not prevent expression of the side effects due to chemotherapy.

Besides these, to prevent and improve side effects of chemotherapy by nutritional intervention, some nutritional supplements are under clinical study. For example, Elental (trade name), which is an elemental diet, has a mucosa-protecting action on glutamine (constituent component), and ingestion thereof during esophageal cancer chemotherapy is known to reduce the onset of stomatitis. In addition, Racol (trade name), which is a low residue diet rich in ω3 fatty acid, has been reported to have a reducing effect on leucopenia, neutropenia, stomatitis and diarrhea in chemical radiation therapy, since ω3 fatty acid has functions such as antiinflammatory action and the like. Also, Prosure (trade name) containing ω3 fatty acid and zinc like Racol has been reported to reduce side effects of chemotherapy.

However, intervention with such nutritional supplements during chemotherapy is generally considered to be poor in compliance, since ingestion of about 400 - 500 ml per day is necessary to achieve an intervention effect, even though the patients have low appetite due to the side effects.

Other approaches taught in the literature are as follows:
WO 2010/033424 discloses immunonutritional compositions for transiently preventing or moderating, anti-cancer treatment induced bone marrow paralysis or neutropenia.
WO 01/80832 discloses methods for treating or mitigating the side effects of cytotoxic cancer therapy for tumours located in the brain, head or neck, comprising administering a thiol-based chemoprotectant agent (for example, N-acetyl cysteine) intra-arterially at a site in the descending aorta or further downstream from the descending aorta, and a cytotoxic agent.
WO 02/41837 discloses therapeutic compositions for treating mucositis at a mucosal site, comprising a pharmaceutical substance effective for preventing or treating mucositis (for example, N-acetylcysteine or glutathione), and a biocompatible polymer.

### SUMMARY OF THE INVENTION

### Problems to be Solved by the Invention

An object of the present invention is to provide an agent for reducing side effects of cancer chemotherapy, which can reduce the above-mentioned various side effects of cancer chemotherapy and improve the treatment completion rate of cancer chemotherapy.

### Means of Solving the Problems

The present inventors have found that ingestion of cystine or a derivative thereof, and theanine in combination can reduce various side effects of cancer chemotherapy and improve the treatment completion rate of cancer chemotherapy, which resulted in the completion of the present invention.

Accordingly, the present invention is as described below.
[1] An agent for use in reducing a side effect of cancer chemotherapy, comprising a combination of:
   (A) cystine or a derivative thereof selected from glutathione, glutathione disulfide, glutathione alkyl ester, oxidized glutathione dialkyl ester, cysteine, cysteine alkyl ester, 3-[(carboxymethyl)thio]alanine, N-acylcysteine, N-acylcysteine alkyl ester, N-acylcystine, N-acylcystine alkyl ester, N,N'-diacylcystine, N,N'-diacylcystine dialkyl ester, or S-alkylcysteine sulfoxide, and a salt of cystine or said derivative thereof; and
   (B) theanine or a salt thereof.
[2] The agent for use according to [1], which is a composition comprising (A) cystine or a derivative thereof and (B) theanine.
[3] The agent for use according to [1] or [2], wherein the weight ratio of (A) cystine or a derivative thereof and (B) theanine is A:B=100:1 - 1:100.
[4] The agent for use according to any one of [1] to [3], which is an agent for improving the treatment completion rate of cancer chemotherapy.
[5] A prophylactic and/or therapeutic drug for cancer, comprising the agent according to any one of [1] to [3], and an anticancer agent in combination.
[6] A combination of:
   (A) cystine or a derivative thereof selected from glutathione, glutathione disulfide, glutathione alkyl ester, oxidized glutathione dialkyl ester, cysteine, cysteine alkyl ester, 3-[(carboxymethyl)thio]alanine, N-acylcysteine, N-acylcysteine alkyl ester, N-acylcystine, N-acylcystine alkyl ester, N,N'-diacylcystine, N,N'-diacylcystine dialkyl ester, or S-alkylcysteine sulfoxide, and a salt of cystine or said derivative thereof; and
   (B) theanine or a salt thereof;
      for use in reducing a side effect of cancer chemotherapy.
[7] The combination according to [6] for use in improving the treatment completion rate of cancer chemotherapy.
[8] A combination of the combination according to [6] and an anticancer agent for use in preventing and/or treating cancer.

### Effect of the Invention

According to the present invention, an agent for reducing side effects of cancer chemotherapy, which can reduce various side effects of cancer chemotherapy and improve the treatment completion rate of cancer chemotherapy, can be provided.

According to the present invention, moreover, an agent for improving the treatment completion rate of cancer chemotherapy, as well as a prophylactic and/or therapeutic agent for cancer can be provided.

### Brief Description of the Drawings

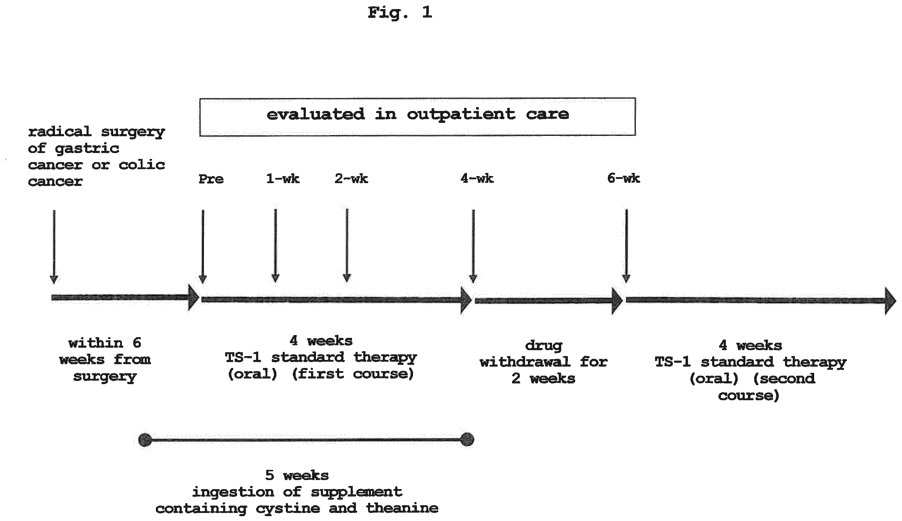

Fig. 1 shows the test schedule of Experimental Example 2.

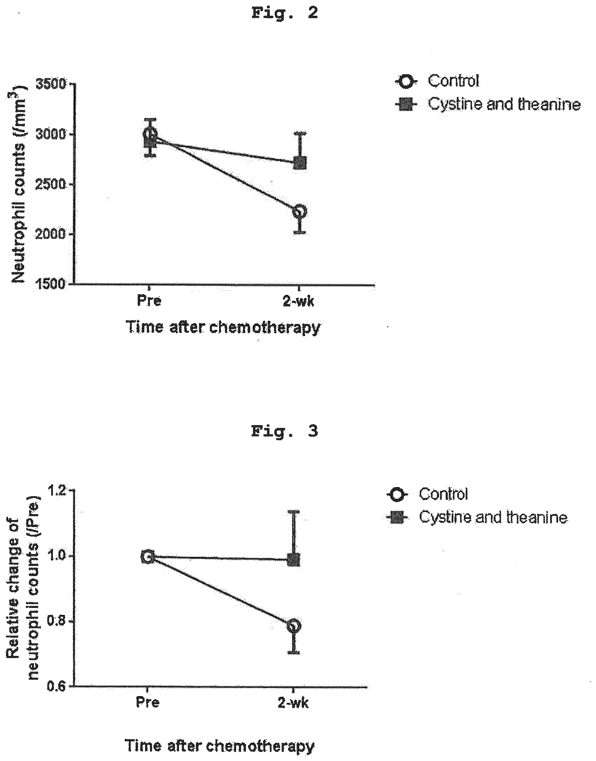

Fig. 2 is a graph showing changes in the number of granulocytes in blood in Experimental Example 2(1-1). The vertical axis shows the number (count) of granulocytes in blood, "Pre" in the horizontal axis shows before administration of a Tegafur-gimeracil-oteracil potassium combination agent, and "2-wk" shows 2 weeks after the start of the administration of the Tegafur-gimeracil-oteracil potassium combination agent.
Fig. 3 is a graph showing changes in the number of granulocytes in blood in Experimental Example 2(1-1). The vertical axis shows the relative value of the number of granulocytes in blood when the number of granulocytes in blood before administration of the Tegafur-gimeracil-oteracil potassium combination agent is 1, "Pre" in the horizontal axis shows before administration of a Tegafur-gimeracil-oteracil potassium combination agent, and "2-wk" shows 2 weeks after the start of the administration of the Tegafur-gimeracil-oteracil potassium combination agent.

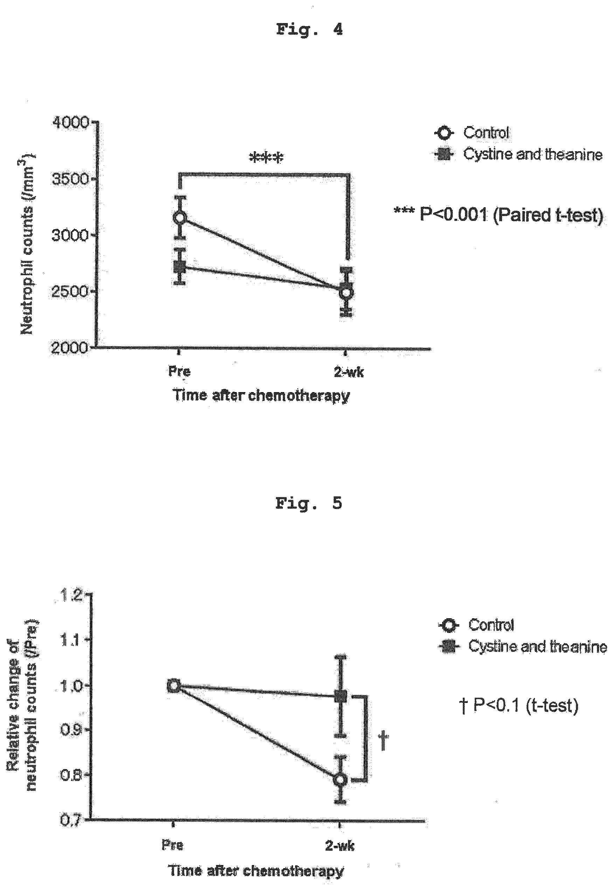

Fig. 4 is a graph showing changes in the number of granulocytes in blood in Experimental Example 2(1-2). The vertical axis shows the number (count) of granulocytes in blood, "Pre" in the horizontal axis shows before administration of a Tegafur-gimeracil-oteracil potassium combination agent, and "2-wk" shows 2 weeks after the start of the administration of the Tegafur-gimeracil-oteracil potassium combination agent. ***: corresponding t-test, p<0.001.
Fig. 5 is a graph showing changes in the number of granulocytes in blood in Experimental Example 2(1-2). The vertical axis shows the relative value of the number of granulocytes in blood when the number of granulocytes in blood before administration of the Tegafur-gimeracil-oteracil potassium combination agent is 1, "Pre" in the horizontal axis shows before administration of a Tegafur-gimeracil-oteracil potassium combination agent, and "2-wk" shows 2 weeks after the start of the administration of the Tegafur-gimeracil-oteracil potassium combination agent. †: t-test, p<0.1.

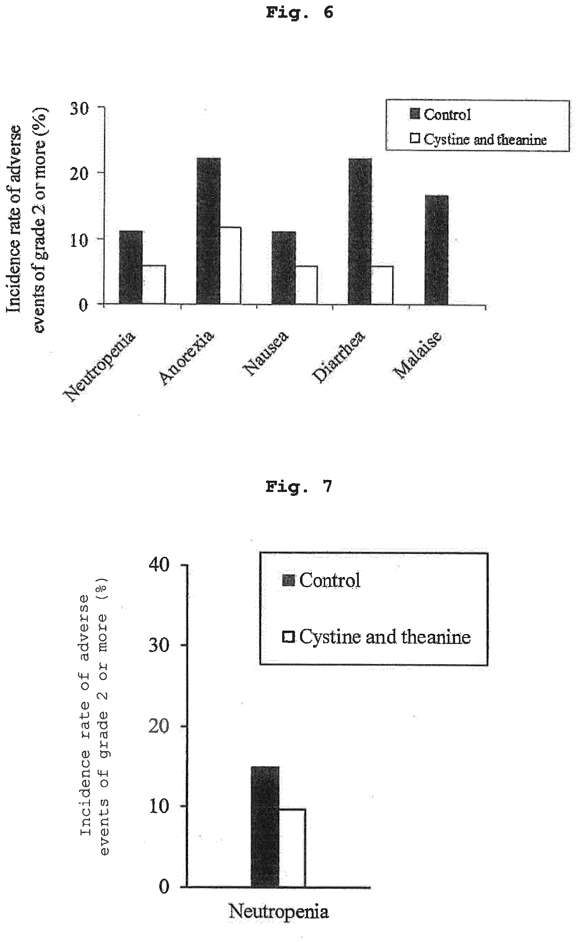

Fig. 6 is a graph showing the incidence of side effects of cancer chemotherapy using a Tegafur-gimeracil-oteracil potassium combination agent in Experimental Example 2(2-1). The vertical axis shows the incidence (%) of each side effect (grade 2 or more) and the horizontal axis shows granulocytopenia, anorexia, nausea, diarrhea, malaise in this order from the left, "Control" shows a control group, and "Cystine and theanine" shows a cystine and theanine ingestion group.
Fig. 7 is a graph showing the incidence of side effects of cancer chemotherapy using a Tegafur-gimeracil-oteracil potassium combination agent in Experimental Example 2(2-2). The vertical axis shows the incidence (%) of side effect (grade 2 or more) and the horizontal axis shows granulocytopenia, "Control" shows a control group, and "Cystine and theanine" shows a cystine and theanine ingestion group.

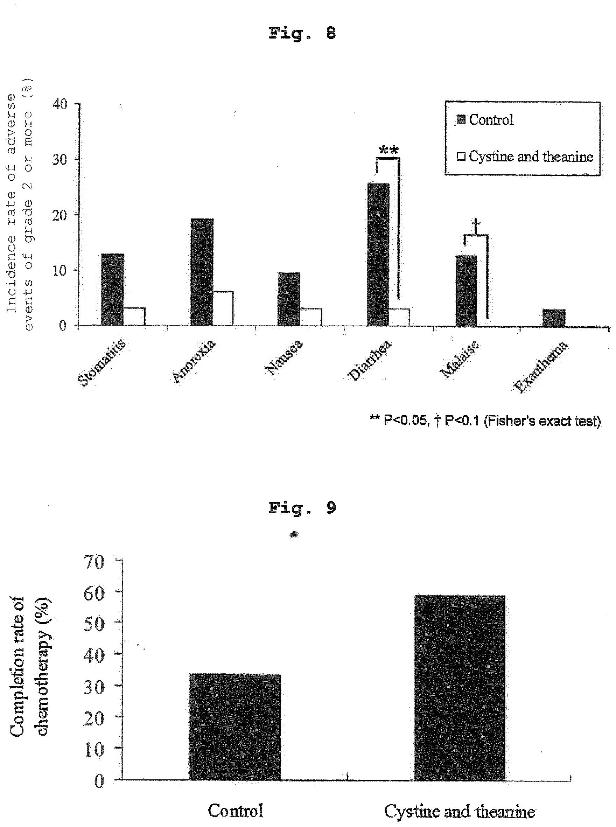

Fig. 8 is a graph showing the incidence of side effects of cancer chemotherapy using a Tegafur-gimeracil-oteracil potassium combination agent in Experimental Example 2(2-2). The vertical axis shows the incidence (%) of each side effect (grade 2 or more) and the horizontal axis shows stomatitis, anorexia, nausea, diarrhea, malaise, exanthema in this order from the left, "Control" shows a control group, and "Cystine and theanine" shows a cystine and theanine ingestion group. **: Fisher's exact test, p<0.05, †: Fisher's exact test, p<0.1.
Fig. 9 is a graph showing the treatment completion rate of cancer chemotherapy using a Tegafur-gimeracil-oteracil potassium combination agent in Experimental Example 2(3-1). The vertical axis shows the completion rate (%) of treatment, "Control" on the horizontal axis shows a control group, and "Cystine and theanine" shows a cystine and theanine ingestion group.

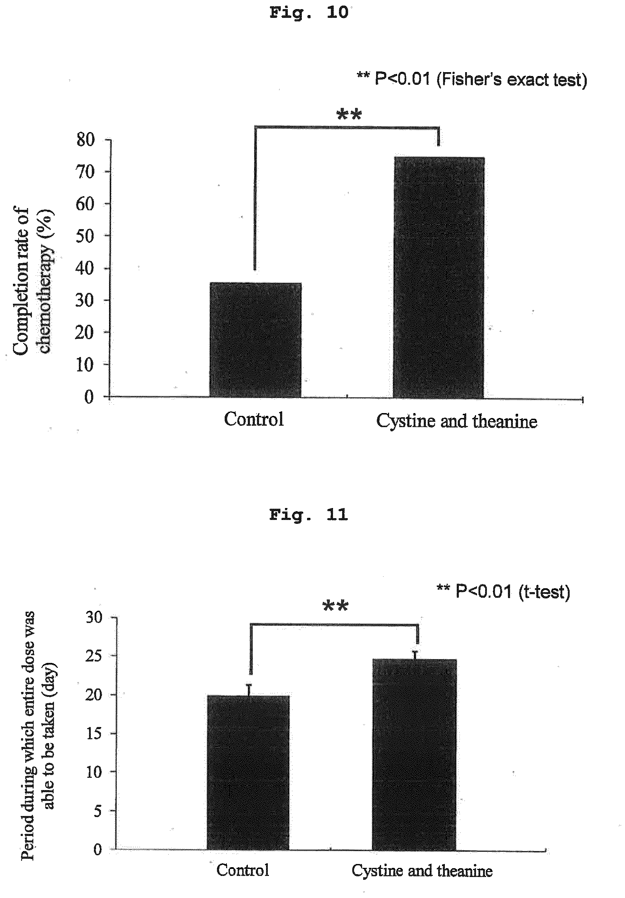

Fig. 10 is a graph showing the treatment completion rate of cancer chemotherapy using a Tegafur-gimeracil-oteracil potassium combination agent in Experimental Example 2(3-2). The vertical axis shows the completion rate (%) of treatment, "Control" on the horizontal axis shows a control group, and "Cystine and theanine" shows a cystine and theanine ingestion group. **: Fisher's exact test, p<0.01.
Fig. 11 is a graph showing the period when the whole dose of the Tegafur-gimeracil-oteracil potassium combination agent could be administered in Experimental Example 2(4). The vertical axis shows the period (days) when the whole dose could be administered, "Control" on the horizontal axis shows a control group, and "Cystine and theanine" shows a cystine and theanine ingestion group. **: t-test, p<0.01.

### Description of Embodiments

### 1. Agent for reducing side effects of cancer chemotherapy

The agent for reducing side effects of cancer chemotherapy of the present invention is mainly characterized in that (A) cystine or a derivative thereof and (B) theanine are used in combination.

In the present specification, the agent for reducing side effects of cancer chemotherapy of the present invention is also referred to as "the agent of the present invention" for convenience.

### [Component A]

Component A in the agent of the present invention is cystine or a derivative thereof.

Any of L-form, D-form and DL-form of cysteine can be used, and preferred is L-form or DL-form, and more preferred is L-form.

The derivative of cystine (cystine derivative) may be any of the compounds listed in claim 1.

The cystine derivative also includes cysteine, which is a monomer of cystine, and compounds derived from cysteine (cysteine derivatives).

The cystine derivative is selected from glutathione, glutathione disulfide (oxidized glutathione), glutathione alkyl ester (e.g., glutathione ethyl ester etc.), oxidized glutathione dialkyl ester (e.g., oxidized glutathione diethyl ester etc.), cysteine, cysteine alkyl ester (e.g., cysteine methyl ester, cysteine ethyl ester etc.), 3-[(carboxymethyl)thio]alanine, N-acylcysteine (e.g., N-acetylcysteine etc.), N-acylcysteine alkyl ester (e.g., N-acetylcysteine methyl ester, N-acetylcysteine ethyl ester etc.), N-acylcystine (e.g., N-acetylcystine etc.), N-acylcystine alkyl ester (e.g., N-acetylcystine methyl ester etc.), N,N'-diacylcystine (e.g., N,N'-diacetylcystine etc.), N,N'-diacylcystine dialkyl ester (e.g., N,N'-diacetylcystine dimethyl ester, N,N'-diacetylcystine diethyl ester etc.), S-alkylcysteine sulfoxide and the like.

The cystine and cystine derivative may take the form of a salt and the term "cystine and cystine derivative" in the present specification conceptually also encompasses salts.

Such salt is not particularly limited as long as it is pharmacologically acceptable, and examples thereof include salts with inorganic acid or organic acid. Examples of the inorganic acid include hydrochloric acid, hydrobromic acid, nitric acid, sulfuric acid, phosphoric acid and the like, and examples of the organic acid include formic acid, acetic acid, trifluoroacetic acid, propionic acid, lactic acid, tartaric acid, oxalic acid, fumaric acid, maleic acid, citric acid, malonic acid, methanesulfonic acid and the like. In addition, it can be a salt with a base. Examples of the salt with a base include alkali metal salts such as sodium salt, potassium salt and the like, alkaline earth metal salts such as calcium salt, magnesium salt and the like, and the like.

Component A is preferably L-cystine.

### [Component B]

Component B in the agent of the present invention is theanine.

Any of L-form, D-form and DL-form of theanine can be used, and preferred is L-form or DL-form, and more preferred is L-form.

The theanine may take the form of a salt and the term "theanine" in the present specification is also a concept encompassing salts.

Such salt is not particularly limited as long as it is pharmacologically acceptable, and examples thereof include salts with inorganic acid or organic acid. Examples of the inorganic acid include hydrochloric acid, hydrobromic acid, nitric acid, sulfuric acid, phosphoric acid and the like, and examples of the organic acid include formic acid, acetic acid, trifluoroacetic acid, propionic acid, lactic acid, tartaric acid, oxalic acid, fumaric acid, maleic acid, citric acid, malonic acid, methanesulfonic acid and the like. In addition, it can be a salt with a base. Examples of the salt with a base include alkali metal salts such as sodium salt, potassium salt and the like, alkaline earth metal salts such as calcium salt, magnesium salt and the like, and the like.

Component B is preferably L-theanine.

The form of the agent of the present invention is not particularly limited as long as component A and component B can be combined on ingestion. For example, the agent of the present invention may be a single composition containing component A and component B. Alternatively, as the agent of the present invention, the first composition containing component A and the second composition containing component B may be used in combination.

The ingestion form of the agent of the present invention is not particularly limited and, for example, (1) the agent may be ingested as a composition containing component A and component B, i.e., single composition, (2) the first composition containing component A and the second composition containing component B may be simultaneously ingested by the same route, (3) the first composition containing component A and the second composition containing component B may be ingested by the same route with time difference (e.g., ingestion in the order of component A and component B, or in the reversed order), (4) the first composition containing component A and the second composition containing component B may be simultaneously ingested by different routes, (5) the first composition containing component A and the second composition containing component B may be ingested by different routes with time difference (e.g., ingestion in the order of component A, component B, or in the reversed order).

When component A and component B are ingested with time difference, the both are preferable co-present in the body.

The weight ratio of component A and component B in the agent of the present invention is generally within the range of A:B=100:1 - 1:100, preferably A:B=20:1 - 1:2 whether they are contained in a single composition or separate compositions.

The ingestion amount of component A in the agent of the present invention per day is generally 1 µg/kg body weight - 800 mg/kg body weight, preferably 100 µg/kg body weight - 100 mg/kg body weight.

In the agent of the present invention, the ingestion amount of component B per day is generally 1 µg/kg body weight - 800 mg/kg body weight, preferably 20 µg/kg body weight - 100 mg/kg body weight.

In the agent of the present invention, the total ingestion amount of component A and component B per day is generally 100 µg/kg body weight - 800 mg/kg body weight, preferably 500 µg/kg body weight - 100 mg/kg body weight.

The daily ingestion amount of the agent of the present invention can be taken at one time or in several times. The ingestion period is not particularly limited, and generally 1 - 90 days, preferably 1 - 30 days.

Examples of the application target of the agent of the present invention include mammals (e.g., human, mouse, rat, hamster, rabbit, cat, dog, bovine, sheep, monkey etc.) and the like, preferably human.

The dosage form of the agent of the present invention is not particularly limited, and examples thereof include oral medicaments such as tablet, granule, powder, capsule (including soft capsule), elixir, syrup, microcapsule, drink , emulsion, suspension and the like; parenteral medicaments such as skin external preparation (e.g., ointment, cream, gel, liquid, lotion, facial mask, bath additive etc.), injection, nasal liquid, infusion and the like; powder product, granular product, capsule product, tablet product, liquid product, jelly product, gum product, sheet product, solid product and the like.

When the agent of the present invention uses the first composition containing component A and the second composition containing component B in combination, the dosage form of these compositions may be the same or different.

The agent of the present invention can contain substances generally used for preparations as necessary. Examples of the substance for preparation include binder, excipient, swelling agent, lubricant, flowability improver, gloss agent, sweetener, flavor, preservative, coating agent, carrier, stabilizer and the like. Additionally, the agent may contain various vitamins and various amino acids.

When the agent of the present invention uses the first composition containing component A and the second composition containing component B in combination, these compositions may contain the same or different substances for preparations.

Examples of the binder include tragacanth, gum arabic, cornstarch, gelatin and the like.

Examples of the excipient include micro-crystalline cellulose, crystalline cellulose and the like.

Examples of the swelling agent include cornstarch, pregelatinized starch, alginic acid, dextrin and the like.

Examples of the lubricant include magnesium stearate and the like.

Examples of the flowability improver include silicon dioxide microparticle and the like.

Examples of the gloss agent include glycerin fatty acid ester and the like.

Examples of the sweetener include sucrose, lactose, aspartame and the like.

Examples of the flavor include peppermint, vanilla flavor, cherry flavor, orange flavor and the like.

Preservative examples of the include methylparaben, propylparaben and the like.

Examples of the coating agent include shellac, sugar and the like.

When the dosage form of the agent of the present invention is capsule, for example, a liquid carrier (e.g., fats and oils) and the like can be contained in addition to the above-mentioned materials.

The agent of the present invention is used to reduce side effects of cancer chemotherapy. In the present specification, "reduction" of the side effects of cancer chemotherapy means alleviation (including disappearance) of the side effects or prevention or delaying of the exacerbation of the side effects. The side effect is a concept encompassing those already developed when the agent of the present invention is ingested, and those developed after ingestion. Therefore, "reduction" of the side effects of cancer chemotherapy in the present specification also means suppression of the development of the side effects in addition to those mentioned above.

Examples of the side effects of cancer chemotherapy include anorexia, nausea, vomiting, diarrhea, stomatitis, malaise, bone marrow toxicity (leukocyte, granulocytopenia), skin disorder, hair loss, neuropathy, interstitial pneumonia, hepatopathy, renopathy, cardiac disorder and the like. Of these, the agent of the present invention is preferably used for reducing side effects (e.g., anorexia, nausea, vomiting, diarrhea, stomatitis, malaise, granulocytopenia etc.) that can lead to the suspension of cancer chemotherapy.

Anticancer agent used for cancer chemotherapy is not particularly limited and includes, for example, anticancer antibiotic, platinum preparation, alkylating agent, antimetabolite, plant alkaloid, agent for molecular targetted therapy, hormonal agent and the like.

The anticancer antibiotic (antitumor antibiotic) refers to an antibiotic belonging to the anticancer agent that suppresses proliferation of cancer cells by damaging DNA or RNA relating to the cancer cells (DNA-damaging anticancer agent). Examples of the anticancer antibiotic include anthracycline anticancer agents such as adriamycin, epidoxorubicin, epirubicin, daunorubicin, aclarubicin, pirarubicin, amrubicin, idarubicin and the like; mitomycin C, bleomycin, peplomycin, chromomycin A3, actinomycin D, zinostatin stimalamer and the like.

The platinum preparation refers to an anticancer agent that exhibits a suppressive effect on the proliferation of cancer cells by binding to the N-7-position of guanine and adenine, which are constituent bases of DNA, at two chlorine atom moieties to form a crosslink in the DNA chain, thereby inhibiting DNA synthesis. Examples of the platinum preparation include cisplatin, carboplatin, nedaplatin, oxaliplatin and the like.

The alkylating agent refers to an anticancer agent that exhibits a suppressive effect on the proliferation of cancer cells by cleaving DNA through alkylation. Examples of the alkylating agent include nitrogen mustards such as nitrogen mustard, nitrogen mustard N-oxide, chlorambucil, ifosfamide, cyclophosphamide, melphalan and the like; ethyleneimines such as carboquone, thiotepa and the like; sulfonic acid esters such as busulfan, improsulfan tosylate and the like; nitrosoureas such as nimustine, ranimustine, dacarbazine, procarbazine and the like, temozolomide and the like.

The antimetabolite refers to an anticancer agent that, during division or proliferation of cancer cells, suppresses proliferation by preventing DNA synthesis by using a substance having a chemical structure similar to that of a substance to be the material of nucleic acid, thus inhibiting the metabolism of cancer cells. Examples of the antimetabolite include pyrimidine antimetabolites such as 5-fluorouracil (5FU), tegafur, carmofur, tegafur-uracil, tegafur-gimeracil-oteracil potassium, doxifluridine, floxuridine, cytarabine, enocitabine, gemcitabine and the like; purine antimetabolites such as mercaptopurine, thioguanine, fludarabine phosphate, thioinosine and the like; folic acid antimetabolites such as methotrexate, trimethoprim, pyrimethamine and the like; and the like.

The plant alkaloid includes, for example, vinca alkaloids that inhibit microtubule formation that acts on the division of cancer cells, taxanes that induce abnormal microtubule formation to inhibit division of cancer cells, topoisomerase inhibitor that inhibits topoisomerase that acts on the cleavage and re-binding of DNA in the process of cell division to eradicate cancer cells and the like. Examples of the vinca alkaloids include vinblastine, vincristine, vindesine, vinorelbine and the like. Examples of the taxanes include docetaxel, paclitaxel and the like. Examples of the topoisomerase inhibitor include irinotecan, nogitecan, etoposide, sobuzoxane and the like.

The agent for molecular targetted therapy refers to an anticancer agent having an activity to kill cancer cells by targeting the molecules involved in the proliferation of cancer cells and inhibiting the molecules. While the agent for molecular targetted therapy is not particularly limited, for example, imatinib, gefitinib, erlotinib, vandetanib, sunitinib, sorafenib, rituximab, cetuximab, infliximab, trastuzumab, bevacizumab and the like can be mentioned.

The hormonal agent refers to an anticancer agent that acts on the hormone involved in the division or proliferation of cancer cells. While the hormonal agent is not particularly limited, for example, anastrozole, exemestane, ethynylestradiol, chlormadinone, goserelin, tamoxifen, bicartamide, flutamide, prednisolone, leuprorelin, retrozole and the like can be mentioned.

The agent of the present invention is preferably used for reducing the side effects of cancer chemotherapy using an antimetabolite (more preferably pyrimidine antimetabolite, particularly preferably 5FU, tegafur-gimeracil-oteracil potassium).

The treatment form of cancer chemotherapy is not particularly limited, and examples thereof include combined modality chemotherapy, multiple drug therapy, (postoperative) adjuvant chemotherapy, preoperative chemotherapy and the like.

### 2. Agent for improving treatment completion rate of cancer chemotherapy

The agent of the present invention can be used as an agent for improving the treatment completion rate of cancer chemotherapy, since it can reduce side effects that possibly suspend cancer chemotherapy.

The "treatment completion rate of cancer chemotherapy" in the present specification refers to a ratio of patients who could complete cancer chemotherapy without suspension due to side effects in the patient groups that underwent the cancer chemotherapy. The diagnostic criteria of whether or not the cancer chemotherapy could be completed can be appropriately set according to, for example, the kind of anticancer agent to be used for the cancer chemotherapy, administration schedule and the like. For example, the administration schedule of cancer chemotherapy using a tegafur-gimeracil-oteracil potassium combination drug can be appropriately set according to the age, sex, disease state and the like of the patients. As shown in the below-mentioned Examples, when oral administration of the drug twice per day for 4 consecutive weeks, followed by 2 weeks of cessation of the drug, is one course, and the administration is repeated, the patients who could complete the first one course without suspension of the drug administration due to the side effects can be judged as the patients who could complete the cancer chemotherapy. Since cancer chemotherapy using a Tegafur-gimeracil-oteracil potassium combination agent shows variation in 5 year survival rate depending on the number of courses that could be performed, improvement of the completion rate of treatment is useful for the improvement of life prognosis.

The anticancer agents to be used for cancer chemotherapy are not particularly limited and examples thereof include those similar to the agents recited as examples in the explanation of the agent of the present invention.

The agent of the present invention can be preferably used for improving the treatment completion rate of cancer chemotherapy using antimetabolite (more preferably pyrimidine antimetabolite, particularly preferably 5FU, tegafur-gimeracil-oteracil potassium).

### 3. Prophylactic and/or therapeutic drug for cancer

The present invention also provides a prophylactic and/or therapeutic drug for cancer, whose side effects (preferably, side effects causing suspension of cancer chemotherapy) have been reduced. The prophylactic and/or therapeutic drug for cancer of the present invention is mainly characterized in that the agent of the present invention and an anticancer agent are used in combination.

In the present specification, the prophylactic and/or therapeutic drug for cancer of the present invention is also referred to as "the prophylactic and/or therapeutic drug of the present invention" for convenience.

Examples of the anticancer agent to be used for the prophylactic and/or therapeutic drug of the present invention include those similar to the agents exemplified as the anticancer agents to be used for cancer chemotherapy, in the explanation of the agent of the present invention.

The prophylactic and/or therapeutic drug of the present invention comprises the agent of the present invention and an anticancer agent in combination. The form thereof is not particularly limited as long as they can be combined at the time of administration. For example, the agent of the present invention and an anticancer agent may be formed as a single preparation or separate preparations.

While anticancer agents are generally administered at a given administration interval with washout period for recovery from the side effects and the like, since the agent of the present invention has high safety, it can be administered even during the washout period of anticancer agents, and can be administered at a shorter interval than the administration interval of anticancer agents. For example, an anticancer agent is administered for a given period (e.g., several days - several weeks etc.), and the agent of the present invention can be administered at shorter intervals than the anticancer agent (e.g., every day etc.). Even in such case, the agent of the present invention and an anticancer agent can be said to be combined.

As the prophylactic and/or therapeutic drug of the present invention, the agent of the present invention and an anticancer agent may be administered simultaneously.

In the prophylactic and/or therapeutic drug of the present invention, when the agent of the present invention and an anticancer agent are formulated into separate preparations, the administration routes of the agent of the present invention and the anticancer agent may be the same or different.

In the prophylactic and/or therapeutic drug of the present invention, the dose of the anticancer agent can be appropriately determined for each kind of the anticancer agent, and according to the pathology, age of the patients, administration method and the like.

The subject of administration of the prophylactic and/or therapeutic drug of the present invention is, for example, a subject similar to the subject of application of the agent of the present invention, and a preferable subject is also the same.

The dosage form of the prophylactic and/or therapeutic drug of the present invention is, for example, a dosage form similar to the dosage form when the agent of the present invention is provided as a drug, and a preferable dosage form is also the same.

In the prophylactic and/or therapeutic drug of the present invention, when the agent of the present invention and an anticancer agent are formulated into separate preparations, the dosage form of these preparations may be the same or different.

The prophylactic and/or therapeutic drug of the present invention can contain pharmaceutically acceptable substances for preparations as necessary. Examples thereof include those similar to the examples recited in the explanation of the agent of the present invention.

In the prophylactic and/or therapeutic drug of the present invention, when the agent of the present invention and an anticancer agent are formulated into separate preparations, the substances for preparations contained in these preparations may be the same or different.

Examples of cancer to be the target of the prophylactic and/or therapeutic drug of the present invention include breast cancers (e.g., infiltrative mammary duct cancer, non-infiltrative mammary duct cancer, inflammatory breast cancer), prostatic cancers (e.g., hormone-dependent prostatic cancer, non-hormone-dependent prostatic cancer), pancreatic cancers (e.g., pancreatic duct cancer), gastric cancers (e.g., papillary adenocarcinoma, mucinous adenocarcinoma, adenosquamous carcinoma), lung cancers (e.g., non-small cell lung cancer, small cell lung cancer, malignant mesothelioma), colic cancers (e.g., gastrointestinal interstitial tumor), rectal cancers (e.g., gastrointestinal interstitial tumor), colorectal cancers (e.g., familial colorectal cancer, hereditary non-polyposis colorectal cancer, gastrointestinal interstitial tumor), small intestine cancers (e.g., non-Hodgkin's lymphoma, gastrointestinal interstitial tumor), esophageal cancer, duodenal cancer, cancer of tongue, pharyngeal cancers (e.g., nasopharyngeal cancer, oropharyngeal cancer, hypopharyngeal cancer), salivary gland cancer, brain tumors (e.g., pineal astrocytoma, hairy cell astrocytoma, diffuse astrocytoma, anaplastic astrocytoma), schwannoma, liver cancers (e.g., primary liver cancer, extrahepatic bile duct cancer), renal cancers (e.g., renal cell carcinoma, transitional epithelium cancer of renal pelvis and ureter), gallbladder cancer, bile duct cancer, pancreatic cancer, liver cancer, endometrial cancer, cervical cancer, ovarian cancers (e.g., epithelial ovarian cancer, extragonadal germ cell tumor, ovarian germ cell tumor, low-malignancy ovarian tumor), bladder carcinoma, urethral cancer, skin cancers (e.g., intraocular (ocular) melanoma, Merkel cell cancer), angioma, malignant lymphoma (e.g., reticulosarcoma, lymphosarcoma, Hodgkin's disease), malignant melanoma, thyroid cancers (e.g., thyroid medullary carcinoma), parathyroid cancer, nasal sinus cancer, paranasal sinus cancer, bone tumors (e.g., osteosarcoma, Ewing's sarcoma, uterine sarcoma, soft tissue sarcoma), angiofibroma, retinal sarcoma, penile cancer, testicular tumor, pediatric solid cancers (e.g., Wilms tumor, pediatric renal tumor), Capodi's sarcoma, AIDS-related Capodi's sarcoma, maxillary sinus tumor, fibrous histiocytoma, smooth muscle sarcoma, rhabdomyosarcoma, leukemias (e.g., acute myelocytic leukemia, acute lymphoblastic leukemia) and the like.

The prophylactic and/or therapeutic drug of the present invention shows reduced side effects, and examples of the side effect include those similar to the side effects exemplified as the side effects of cancer chemotherapy in the explanation of the agent of the present invention.

While the present invention is explained in more detail in the following Examples, the present invention is not limited by the Examples.

### Examples

### Production Example 1: Production of supplement containing cystine and theanine

L-cystine (2,625 g), L-theanine (1,050 g), dextrin (1,710 g), aspartame (15 g) were mixed, crystalline cellulose (1,500 g) and 70% ethanol at a weight ratio of 30% were added, and the mixture was kneaded and extrusion-granulated. The obtained granules were dried to a water content of not more than 1.6% and sieved to give 16 mesh PASS particles. The particles were mixed with silicon dioxide microparticles (37.5 g), glycerin fatty acid ester (487.5 g) and vanilla flavor (75 g). The obtained mixture was tableted under the conditions of 11 mmΦ, 500 mg, tableting pressure 2.0 ton, revolution 20 rpm, hardness average 10 kg. By the above operation, a 11 mmΦ tablet containing 175 mg of L-cystine and 70 mg of L-theanine per tablet (total amount: 425 mg) was produced.

### Experimental Example 1: Evaluation of side effects reducing effect in patients who developed stomatitis during cancer chemotherapy using multiple drugs in combination

Cancer chemotherapy by a combined use of multiple drugs was performed, and 14 patients who developed stomatitis as the side effect were used as the subject of the test. The disease of the patients was rectal cancer, colic cancer, gastric cancer or breast cancer. The patients underwent cancer chemotherapy by a combined use of multiple drugs, based on the regimen corresponding to the kind of cancer and progress stage, and ingested a cystine and theanine-containing supplement (700 mg of L-cystine, 280 mg of L-theanine, the total amount of supplement 1.7 g) once per day for one month from the time point when the onset of stomatitis was confirmed by CTCAE v4.0. One month from the start of the ingestion, the condition of stomatitis was evaluated using CTCAE v4.0 grade classification. Also, the condition of stomatitis and pain were subjectively evaluated by the patients themselves. The evaluation criteria of the subjective evaluation were as follows.

### [Condition of stomatitis]

1: healed or no occurrence
2: became better though not completely recovered
3: no change
4: exacerbated somewhat
5: considerably exacerbated

### [Pain of stomatitis]

1: gone
2: became better though not gone
3: no change
4: worsened somewhat
5: considerably worsened

The results are shown in Table 1.

**Table 1**

| patients | | | anticancer agent | evaluation by CTCAE v4.0 | | subjective evaluation by patients themselves | |
|---|---|---|---|---|---|---|---|
| age | sex | disease | | immediately after onset | after ingestion | condition | pain |
| 60 | M | rectal cancer | BV+FOLFILI | 2 | 0 | 1 | 1 |
| 76 | M | colic cancer | cetu+CPT11 | 2 | 1 | 2 | 2 |
| 74 | M | colic cancer | FOLFILI | 2 | 0 | 2 | 1 |
| 64 | M | colic cancer | FOLFILI+BV | 1 | 0 | 1 | 1 |
| 79 | M | colic cancer | FOLFOX | 1 | 0 | 2 | 1 |
| 77 | M | colic cancer | FOLFOX+BV | 1 | 1 | 3 | 3 |
| 45 | F | breast cancer | FEC | 2 | 0 | 1 | 1 |
| 63 | M | colic cancer | FOLFOX+BV>Cmab+CPT11 | 1 | 1 | 2 | 3 |
| 73 | F | rectal cancer | FOLFILI+BV | 1 | 0 | 1 | 1 |
| 54 | F | breast cancer | HPT+DTX>HPT+S1 | 1 | 2 | 2 | 2 |
| 62 | M | rectal cancer | FOLFOX | 1 | 1 | 3 | 3 |
| 58 | F | gastric cancer | TS1+CDDP | 1 | 0 | 1 | 3 |
| 68 | M | rectal cancer | FOLFILI+BV | 1 | 1 | 2 | 2 |
| 61 | F | breast cancer | FEC | 1 | 1 | 2 | 3 |

Note) BV: bevacizumab, FOLIFILI: 5FU+l-Leucovorin+irinotecan, cetu and Cmab: cetuximab, CPT11: irinotecan hydrochloride, FOLFOX: 5FU+l-Leucovorin+oxaliplatin, FEC: 5FU+epirubicin+cyclophosphamide, HPT: herceptin (trade name, trastuzumab), DXT: docetaxel, S1 and TS1: TS-1 (trade name, Tegafur-gimeracil-oteracil potassium combination), CDDP: cisplatin

The "FOLFOX+BV>Cmab+CPT11" means that FOLFOX+BV was initially administered, and thereafter, Cmab+CPT11 was administered instead. Similarly, "HPT+DTX>HPT+S1" means that HPT+DTX was initially administered, and thereafter, HPT+S1 was administered instead.

As shown in Table 1, among the patients who developed stomatitis after the start of cancer chemotherapy by a combined use of multiple drugs and took a supplement containing cystine and theanine for one month, 57.1% of the patients showed improvement of stomatitis by the evaluation according to CTCAE v4.0 side effect grade classification. Also, in the subjective evaluation by patients themselves, 85.7% of patients showed improvement in the condition of stomatitis, and 64.3% of patients showed improvement in the pain due to stomatitis.

From these results, it was clarified that ingestion of a supplement containing cystine and theanine in patients who developed stomatitis after the start of cancer chemotherapy by a combined use of multiple drugs improved stomatitis.

### Experimental Example 2: Evaluation of side effect-reducing effect during postoperative adjuvant chemotherapy in colorectal cancer or gastric cancer

Patients scheduled for administration of a Tegafur-gimeracil-oteracil potassium combination agent (trade name: TS-1) as a postoperative adjuvant chemotherapy for colorectal cancer or gastric cancer, within 6 weeks from the surgical operation, were used as the subjects of the test. The target patients were divided into two groups of a group for ingesting cystine and theanine (cystine and theanine ingestion group), and a group for not ingesting cystine and theanine (control group) by the envelope method. In this test, the dose of a Tegafur-gimeracil-oteracil potassium combination agent was set to a tegafur equivalent amount of 80 mg/day for patients with a body surface area of less than 1.25 m², a tegafur equivalent amount of 100 mg/day when the body surface area was 1.25 m² or more - less than 1.5 m², and a tegafur equivalent amount of 120 mg/day when the body surface area was 1.5 m² or more. The dose was appropriately adjusted according to the kidney function (creatinine clearance) of the patients. As the administration schedule of a Tegafur-gimeracil-oteracil potassium combination agent, the combination agent was orally administered twice per day every day for 4 consecutive weeks, and the drug was withdrawn for 2 weeks. Using this as one course, the administration was repeated. The cystine and theanine ingestion group ingested a cystine and theanine-containing supplement (700 mg of L-cystine, 280 mg of L-theanine, the total amount of supplement 1.7 g) once per day in the morning together with a small amount of water for 5 continuous weeks (including 4 weeks of the first course of the administration of a Tegafur-gimeracil-oteracil potassium combination agent) from one week before the start of the administration of the Tegafur-gimeracil-oteracil potassium combination agent. The control group ingested only a Tegafur-gimeracil-oteracil potassium combination agent, without ingesting the cystine and theanine containing supplement. The both groups were inhibited from taking supplements and vitamins until the completion of the test. Blood samples were collected and symptoms (anorexia, nausea, diarrhea and malaise) of side effects were monitored before administration of a Tegafur-gimeracil-oteracil potassium combination agent, and 1 week, 2 weeks, 4 weeks and 6 weeks after the start of the administration. The results of blood samples, development of side effects and severity thereof were evaluated using the CTCAE v4.0 grade classification. In the evaluation based on the CTCAE v4.0 grade classification, administration of a Tegafur-gimeracil-oteracil potassium combination agent was discontinued in patients of grade 2 or more in any of the number of granulocytes in blood, anorexia, nausea, diarrhea and malaise. Fig. 1 depicts the test schedule.

(1-1) The cystine and theanine ingestion group (14 subjects) and the control group (15 subjects) were analyzed for granulocytopenia associated with bone marrow toxicity by the administration of a Tegafur-gimeracil-oteracil potassium combination agent.

The results are shown in Fig. 2 and Fig. 3.

As shown in Fig. 2 and Fig. 3, granulocytopenia associated with bone marrow toxicity was confirmed in the control group at 2 weeks after the start of administration of a Tegafur-gimeracil-oteracil potassium combination agent; however, granulocytopenia was suppressed in the cystine and theanine ingestion group.

From the results, it was clarified that the ingestion of a supplement containing cystine and theanine reduced granulocytopenia associated with administration of a Tegafur-gimeracil-oteracil potassium combination agent.

(1-2) After the analysis of the above-mentioned (1-1), the number of the cystine and theanine ingestion group was increased from 14 to 25, and the number of the control group was increased from 15 to 25, and they were analyzed for granulocytopenia associated with bone marrow toxicity by the administration of a Tegafur-gimeracil-oteracil potassium combination agent, in the same manner as in the above-mentioned (1-1).

The results are shown in Fig. 4 and Fig. 5.

As shown in Fig. 4 and Fig. 5, granulocytopenia associated with bone marrow toxicity was confirmed in the control group at 2 weeks after the start of administration of a Tegafur-gimeracil-oteracil potassium combination agent; however, granulocytopenia was suppressed in the cystine and theanine ingestion group.

From the results, therefore, it was also confirmed that the ingestion of a supplement containing cystine and theanine reduced granulocytopenia associated with administration of a Tegafur-gimeracil-oteracil potassium combination agent.

(2-1) The cystine and theanine ingestion group (17 subjects) and the control group (18 subjects) were evaluated for each side effect associated with the administration of a Tegafur-gimeracil-oteracil potassium combination agent, by using CTCAE v4.0 grade classification. To be specific, side-effect incidence of grade 2 or more, which is the drug cessation standard of TS-1, was analyzed for granulocytopenia, anorexia, nausea, diarrhea and malaise.

The results are shown in Fig. 6.

As shown in Fig. 6, the incidence decreased in the cystine and theanine ingestion group as compared to the control group, in any of granulocytopenia, anorexia, nausea, diarrhea and malaise.

From the results, it was clarified that the ingestion of a supplement containing cystine and theanine reduced the onset of granulocytopenia, anorexia, nausea, diarrhea and malaise associated with administration of a Tegafur-gimeracil-oteracil potassium combination agent.

(2-2) After the analysis of the above-mentioned (2-1), the number of the cystine and theanine ingestion group and that of the control group were increased, and they were evaluated for each side effect associated with the administration of a Tegafur-gimeracil-oteracil potassium combination agent, by using CTCAE v4.0 grade classification. To be specific, granulocytopenia in 31 subjects of the cystine and theanine ingestion group and 27 subjects of the control group, and stomatitis, anorexia, nausea, diarrhea, malaise and exanthema in 32 subjects of the cystine and theanine ingestion group and 31 subjects of the control group were analyzed for the side-effect incidence of grade 2 or more.

The results are shown in Fig. 7 and Fig. 8.

As shown in Fig. 7 and Fig. 8, the incidence decreased in the cystine and theanine ingestion group as compared to the control group, in any of granulocytopenia, stomatitis, anorexia, nausea, diarrhea, malaise and exanthema.

From the results, it was clarified that the ingestion of a supplement containing cystine and theanine reduced the onset of granulocytopenia, stomatitis, anorexia, nausea, diarrhea, malaise and exanthema associated with administration of a Tegafur-gimeracil-oteracil potassium combination agent.

(3-1) The cystine and theanine ingestion group (17 subjects) and the control group (18 subjects) were analyzed for the treatment completion rate of cancer chemotherapy using a Tegafur-gimeracil-oteracil potassium combination agent. The number (N) of patients who could complete the first one course of the cancer chemotherapy without suspending the administration of a Tegafur-gimeracil-oteracil potassium combination agent was taken as the "treatment completion case number", and the ratio (%) of the treatment completion case number to the total number of patients in each group (cystine and theanine ingestion group: 17 subjects, control group: 18 subjects) was calculated as the "completion rate of treatment".

The results are shown in Table 2 and Fig. 9.

**Table 2**

| | control group (N=18) | cystine and theanine ingestion group (N=17) |
|---|---|---|
| treatment completion case number (N) | 6 | 10 |
| completion rate of treatment (%) | 33.33 | 58.82 |

As shown in Table 2 and Fig. 9, the value of the completion rate of treatment was high in the cystine and theanine ingestion group as compared to the control group.

From the results, it was clarified that the ingestion of a supplement containing cystine and theanine reduced various side effects associated with administration of a Tegafur-gimeracil-oteracil potassium combination agent and improved the treatment completion rate of cancer chemotherapy.

(3-2) After the analysis of the above-mentioned (3-1), the number of the cystine and theanine ingestion group was increased from 17 to 32, and the number of the control group was increased from 18 to 31, and they were analyzed for the treatment completion rate of cancer chemotherapy using a Tegafur-gimeracil-oteracil potassium combination agent, in the same manner as in the above-mentioned (3-1).

The results are shown in Table 3 and Fig. 10.

**Table 3**

| | control group (N=31) | cystine and theanine ingestion group (N=32) |
|---|---|---|
| treatment completion case number (N) | 11 | 24 |
| completion rate of treatment (%) | 35.5 | 75.0 |

As shown in Table 3 and Fig. 10, the value of the completion rate of treatment was high in the cystine and theanine ingestion group as compared to the control group.

Also, from the results, therefore, it was confirmed that the ingestion of a supplement containing cystine and theanine reduced various side effects associated with administration of a Tegafur-gimeracil-oteracil potassium combination agent and improved the treatment completion rate of cancer chemotherapy.

(4) The cystine and theanine ingestion group (32 subjects) and the control group (31 subjects) were analyzed for the period when the whole dose of a Tegafur-gimeracil-oteracil potassium combination agent could be taken without dose reduction due to the side effects. In this test, the dose of a Tegafur-gimeracil-oteracil potassium combination agent was withdrawn or reduced basically when side effects of grade 2 or more in the CTCAE v4.0 grade classification were expressed, according to the package insert of the combination agent.

The results are shown in Fig. 11.

As shown in Fig. 11, the period when the whole dose of a Tegafur-gimeracil-oteracil potassium combination agent could be taken was longer in the cystine and theanine ingestion group as compared to the control group.

From the results, it was clarified that the ingestion of a supplement containing cystine and theanine reduced various side effects associated with administration of a Tegafur-gimeracil-oteracil potassium combination agent and the whole dose of the combination agent could be taken for a longer term.

### Industrial Applicability

According to the present invention, an agent for reducing side effects of cancer chemotherapy, which can reduce various side effects of cancer chemotherapy and improve the treatment completion rate of cancer chemotherapy, can be provided.

According to the present invention, moreover, an agent for improving the treatment completion rate of cancer chemotherapy, as well as a prophylactic and/or therapeutic agent for cancer can be provided.

## Claims

1. An agent for use in reducing a side effect of cancer chemotherapy, comprising a combination of:
(A) cystine or a derivative thereof selected from glutathione, glutathione disulfide, glutathione alkyl ester, oxidized glutathione dialkyl ester, cysteine, cysteine alkyl ester, 3-[(carboxymethyl)thio]alanine, N-acylcysteine, N-acylcysteine alkyl ester, N-acylcystine, N-acylcystine alkyl ester, N,N'-diacylcystine, N,N'-diacylcystine dialkyl ester, or S-alkylcysteine sulfoxide, and a salt of cystine or said derivative thereof; and
(B) theanine or a salt thereof.

2. The agent for use according to claim 1, which is a composition comprising (A) cystine or a derivative thereof and (B) theanine.

3. The agent for use according to claim 1 or 2, wherein the weight ratio of (A) cystine or a derivative thereof and (B) theanine is A:B=100:1 - 1:100.

4. The agent for use according to any one of claims 1 to 3, which is an agent for improving the treatment completion rate of cancer chemotherapy.

5. A prophylactic and/or therapeutic drug for cancer, comprising the agent according to any one of claims 1 to 3, and an anticancer agent in combination.

6. A combination of:
(A) cystine or a derivative thereof selected from glutathione, glutathione disulfide, glutathione alkyl ester, oxidized glutathione dialkyl ester, cysteine, cysteine alkyl ester, 3-[(carboxymethyl)thio]alanine, N-acylcysteine, N-acylcysteine alkyl ester, N-acylcystine, N-acylcystine alkyl ester, N,N'-diacylcystine, N,N'-diacylcystine dialkyl ester, or S-alkylcysteine sulfoxide, and a salt of cystine or said derivative thereof; and
(B) theanine or a salt thereof;
for use in reducing a side effect of cancer chemotherapy.

7. The combination according to claim 6 for use in improving the treatment completion rate of cancer chemotherapy.

8. A combination of the combination according to claim 6 and an anticancer agent for use in preventing and/or treating cancer.

## Patentansprüche

1. Mittel zur Verwendung bei der Verringerung einer Nebenwirkung einer Krebs-Chemotherapie, umfassend eine Kombination aus
(A) Cystin oder eineim Derivat davon, ausgewählt aus Glutathion, Glutathiondisulfid, Glutathionalkylester, oxidiertem Glutathiondialkylester, Cystein, Cysteinalkylester, 3-[(Carboxymethyl)thio]alanin, N-Acylcystein, N-Acylcysteinalkylester, N-Acylcystein, N-Acylcysteinalkyl-10-ester, N,N'-Diacylcystin, N,N'-Diacylcystindialkylester oder S-Alkylcysteinsulfoxid und einem Salz von Cystin oder dessen Derivat; und
(B) Theanin oder einem Salz davon.

2. Mittel zur Verwendung nach Anspruch 1, das eine (A) Cystin oder ein Derivat davon und (B) Theanin umfassende Zusammensetzung ist.

3. Mittel zur Verwendung nach Anspruch 1 oder 2, wobei das 20-Gewichtsverhältnis von (A) Cystin oder einem Derivat davon und (B) Theanin A:B= 100:1 - 1:100 beträgt.

4. Mittel zur Verwendung nach einem der Ansprüche 1 bis 3, das ein Mittel zur Verbesserung der Behandlungsabschlussrate von Krebs-Chemotherapien.

5. Prophylaktisches und/oder therapeutisches Arzneimittel gegen Krebs, umfassend das Mittel nach einem der Ansprüche 1 bis 3 und ein Antikrebsmittel in Kombination.

6. Kombination aus:
(A) Cystin oder einem Derivat davon, ausgewählt aus Glutathion, Glutathiondisulfid, Glutathionalkylester, oxidiertem Glutathiondialkylester, Cystein, Cysteinalkylester, 3-[(Carboxymethyl)thio]alanin, N-Acylcystein, N-Acylcysteinalkylester, N-Acylcystein, N-Acylcysteinalkylester, N,N'-Diacylcystin, N,N'-Diacylcystindialkylester oder S-Alkylcysteinsulfoxid und einem Salz von Cystin oder dessen Derivat; und
(B) Theanin oder einem Salz davon;
zur Verwendung bei der Verringerung einer Nebenwirkung der Krebs-Chemotherapie.

7. Kombination nach Anspruch 6 zur Verwendung bei der Verbesserung der Behandlungsabschlussrate von Krebs-Chemotherapien.

8. Kombination aus der Kombination nach Anspruch 6 und einem Antikrebsmittel zur Verwendung bei der Prävention und/oder Behandlung von Krebs.

## Revendications

1. Agent pour une utilisation pour atténuer un effet secondaire de la chimiothérapie du cancer, comprenant une combinaison de :
(A) cystine ou un dérivé de celle-ci sélectionné parmi glutathion, disulfure de glutathion, alkylester de glutathion, dialkylester de glutathion oxydé, cystéine, alkylester de cystéine, 3-[(carboxyméthyl)thio]alanine, N-acylcystéine, alkylester de N-acylcystéine, N-acylcystine, alkylester de N-acylcystine, N,N'-diacylcystine, dialkylester de N,N'-diacylcystine ou sulfoxyde de S-alkylcystéine, et un sel de cystine ou ledit dérivé de celui-ci ; et
(B) théanine ou un sel de celle-ci.

2. Agent pour une utilisation selon la revendication 1, lequel est une composition comprenant (A) cystine ou un dérivé de celle-ci et (B) théanine.

3. Agent pour une utilisation selon la revendication 1 ou 2, dans lequel le rapport en poids de (A) cystine ou un dérivé de celle-ci et (B) théanine est A:B=100:1 - 1:100.

4. Agent pour une utilisation selon l'une quelconque des revendications 1 à 3, lequel est un agent pour améliorer le taux d'achèvement de traitement de la chimiothérapie du cancer.

5. Médicament prophylactique et/ou thérapeutique pour le cancer, comprenant l'agent selon l'une quelconque des revendications 1 à 3 et un agent anticancéreux en combinaison.

6. Combinaison de :
(A) cystine ou un dérivé de celle-ci sélectionné parmi glutathion, disulfure de glutathion, alkylester de glutathion, dialkylester de glutathion oxydé, cystéine, alkylester de cystéine, 3-[(carboxyméthyl)thio]alanine, N-acylcystéine, alkylester de N-acylcystéine, N-acylcystine, alkylester de N-acylcystine, N,N'-diacylcystine, dialkylester de N,N'-diacylcystine ou sulfoxyde de S-alkylcystéine, et un sel de cystine ou ledit dérivé de celui-ci ; et
(B) théanine ou un sel de celle-ci ;
pour une utilisation pour atténuer un effet secondaire de la chimiothérapie du cancer.

7. Combinaison selon la revendication 6 pour une utilisation pour améliorer le taux d'achèvement de traitement de la chimiothérapie du cancer.

8. Combinaison de la combinaison selon la revendication 6 et d'un agent anticancéreux pour une utilisation pour prévenir et/ou traiter le cancer.
